(19) **Europäisches Patentamt**
**European Patent Office**
**Office européen des brevets**

(11) **EP 2 997 381 B1**

(12) **FASCICULE DE BREVET EUROPEEN**

(45) Date de publication et mention
de la délivrance du brevet:
**11.04.2018   Bulletin 2018/15**

(51) Int Cl.:
*G01N 33/68* (2006.01)        *G06F 19/24* (2011.01)
*G01N 33/543* (2006.01)

(21) Numéro de dépôt: **14731684.8**

(22) Date de dépôt: **12.05.2014**

(86) Numéro de dépôt international:
**PCT/FR2014/051086**

(87) Numéro de publication internationale:
**WO 2014/184476 (20.11.2014 Gazette 2014/47)**

(54) **PROCEDE DE MESURE DE LA CONCENTRATION PLASMATIQUE D'UN ANALYTE DIRECTEMENT SUR UN ECHANTILLON DE SANG TOTAL**

VERFAHREN ZUR MESSUNG DER PLASMAKONZENTRATION EINES ANALYTEN DIREKT AUF EINER VOLLBLUTPROBE

METHOD FOR MEASURING THE PLASMA CONCENTRATION OF AN ANALYTE DIRECTLY ON A WHOLE BLOOD SAMPLE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **13.05.2013   FR 1354276**

(43) Date de publication de la demande:
**23.03.2016   Bulletin 2016/12**

(73) Titulaire: **Biomérieux**
**69280 Marcy l'Étoile (FR)**

(72) Inventeurs:
- **CHEUCLE, Sylvie**
**F-69890 La Tour de Salvagny (FR)**
- **MARILLET, Laure**
**F-69009 Lyon (FR)**
- **THOLLET, Aurélie**
**F-44000 Nantes (FR)**

(74) Mandataire: **Morel, Anne-Aurore**
**BioMérieux**
**Chemin de l'Orme**
**69280 Marcy l'Etoile (FR)**

(56) Documents cités:
**US-A1- 2009 177 406**

- **LAMERS YVONNE ET AL: "Red blood cell folate concentrations increase more after supplementation with [6S]-5-methyltetrahydrofolate than with folic acid in women of childbearing age", AMERICAN JOURNAL OF CLINICAL NUTRITION, vol. 84, no. 1, juillet 2006 (2006-07), pages 156-161, XP002719609, ISSN: 0002-9165**
- **J. A. STRASESKI ET AL: "Investigating Interferences of a Whole-Blood Point-of-Care Creatinine Analyzer: Comparison to Plasma Enzymatic and Definitive Creatinine Methods in an Acute-Care Setting", CLINICAL CHEMISTRY, vol. 57, no. 11, 15 septembre 2011 (2011-09-15), pages 1566-1573, XP055098981, ISSN: 0009-9147, DOI: 10.1373/clinchem.2011.165480**
- **Correspondence : Dr ET AL: "Letter Letter to the EditorsLetter to the Editors Comparability of whole-blood and plasma clozapine and norclozapine concentrations", Blackwell Publishing Ltd Br J Clin Pharmacol Blackwell Science UK BCPBritish Journal of Clinical Pharmacology, 1 janvier 2003 (2003-01-01), pages 135-138, XP055098980, Extrait de l'Internet: URL:http://www.ncbi.nlm.nih.gov/pmc/articles/PMC1884330/pdf/bcp0056-0135.pdf [extrait le 2014-01-28]**

**Description**

**DOMAINE DE L'INVENTION**

La présente invention a trait à l'analyse d'échantillons biologiques, et plus particulièrement la mesure de la concentration ou de la dose d'un analyte dans un échantillon de sang. L'invention trouve notamment application dans les mesures par immunodosage du type ELISA, ELFA, ou immuno-capture.

**ETAT DE LA TECHNIQUE**

Usuellement, la recherche d'un analyte d'intérêt et de sa concentration dans un échantillon de sang total susceptible de contenir ledit analyte d'intérêt consiste dans un premier temps à séparer le plasma des globules rouges, notamment par centrifugation, puis à réaliser la mesure de la concentration de l'analyte dans le plasma.

Le « sang total » correspond à un échantillon biologique comprenant du sang avec tous ses constituants et donc notamment le plasma et les globules rouges. Il peut par exemple s'agir de sang prélevé sur un homme ou un animal sans aucune transformation, ou de ce sang auquel ont été ajoutés des adjuvants, par exemple des anti-coagulants.

Parmi les méthodes de mesure de la concentration, on connait des techniques consistant à modifier une propriété mesurable, par exemple optique, électrique, chimique, de pH, ou enzymatique, en fonction de la quantité d'analytes dans un échantillon sanguin, notamment les technique dites ELISA (pour « *Enzyme-Linked ImmnoSorben Assay* ») directe, indirecte et par compétition, ELFA (pour « *Enzyme-Linked Fluorescente Assay* ») et d'immuno-capture.

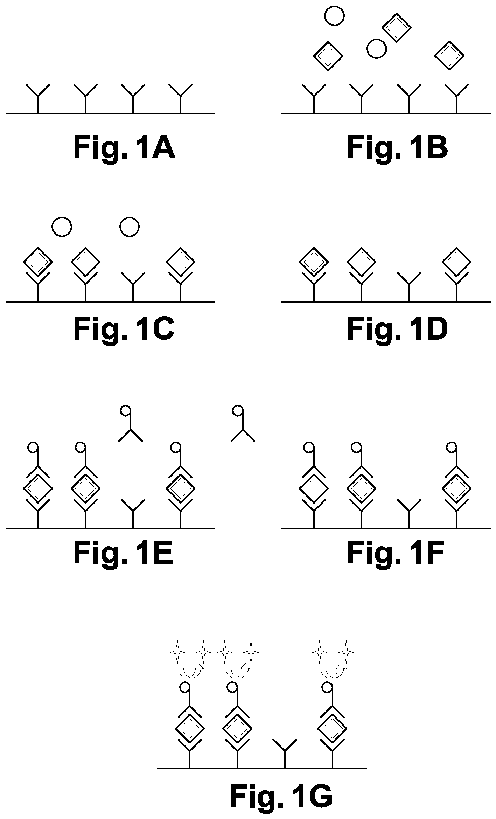

La figure 1 illustre schématiquement les principales étapes d'une mesure ELISA directe, également nommée ELISA « sandwich » car elle met en oeuvre deux partenaires de liaisons à l'analyte, dans laquelle, par exemple, l'analyte est un antigène et les deux partenaires de liaison sont des anticorps comportant chacun un site, ou épitope, différents l'un de l'autre et aptes chacun à se lier audit antigène muni d'un site complémentaire. La mesure ELISA consiste, dans sa version la plus simple, à :

- revêtir la surface d'un support solide, tel qu'un puits par exemple, d'une couche d'un premier partenaire de liaison à l'analyte dont on cherche la présence ou à mesurer la concentration, lequel premier partenaire de liaison présente un site complémentaire à l'analyte (figure 1A);
- à verser l'échantillon sanguin, tel que du plasma issu de la centrifugation d'un échantillon de sang total, dans le puits (figure 1B) de manière à ce que les analytes présents dans le plasma se conjuguent aux premiers partenaires de liaison fixés à la paroi du puits (figure 1C) ;
- à réaliser un premier lavage du puits de manière à éliminer les éléments ne présentant pas d'intérêts pour l'analyse en cours, par exemple les anticorps et antigènes non recherchés (figure 1D) ;
- à verser dans le puits des deuxièmes partenaires de liaison à l'analyte, munis chacun (i) d'un site, complémentaire à l'un des sites libres des analytes immobilisés à l'aide des premiers partenaires de liaison et différent du site complémentaire du premier partenaire de liaison, et (ii) d'un composant présentant une fonction enzymatique apte à catalyser une hydrolyse d'un substrat qui change de couleur en fonction de la quantité d'hydrolyse catalysé (figure 1E). On appelle de tels deuxièmes partenaires de liaison contenant une fonction enzymatiques des « conjugués »;
- à réaliser un second lavage du puits de manière à éliminer les conjugués en excès (figure 1F) ;
- à ajouter du substrat dégradable par la fonction enzymatique des conjugués dans le puits et la mesure de la chrominance ou densité optique du milieu contenu dans le puits par spectrométrique (figure 1G).

La chrominance étant liée directement à la quantité ou dose d'analytes immobilisés par les premiers partenaires de liaison fixés à la paroi du support solide, la mesure de cette propriété optique est ainsi une mesure indirecte de la quantité totale d'analytes présent dans l'échantillon de plasma, et par conséquent, connaissant le volume de l'échantillon, de la concentration d'analyte dans ce dernier. La chrominance mesurée est ensuite transformée, à l'aide d'un modèle mathématique prédéterminé, en une valeur de dose et/ou de concentration. Par « dose », on entend ainsi une quantité d'analytes dans un échantillon. Par « concentration », on entend une dose divisée par le volume de l'échantillon sur lequel est pratiquée la mesure.

S'agissant de la mesure ELISA dite par « compétition », elle met en oeuvre un seul partenaire de liaison à l'analyte qui présente un site complémentaire à l'un des sites de l'analyte, ainsi qu'un composé qui entre en compétition avec l'analyte à doser. L'un de ces deux éléments possède alors une fonction enzymatique apte à catalyser une hydrolyse d'un substrat qui change de couleur en fonction de la quantité d'hydrolyse catalysé. La dose et donc la concentration est alors inversement à la chrominance lue.

Les mesures ELFA sont comparables aux mesures ELISA à ceci près que le substrat catalysé par la fonction enzymatique produit de la fluorescence mesurée par exemple par un fluorimètre.

Dans toutes ces techniques de mesure, il est donc prévu des éléments fixant uniquement les analytes dont on cherche à mesurer la concentration. Bien que seuls ces derniers soient fixés de manière spécifique, on observe cependant que la nature de l'échantillon influe sensiblement sur la mesure de la dose, surtout lorsque le temps de réaction est réduit du fait des contraintes de temps liées au diagnostic. Ainsi, en réalisant la mesure directement sur un échantillon de sang total, la dose mesurée est inférieure à celle directement mesurée sur un échantillon de plasma, notamment en raison de la présence d'hématocrites qui a un effet matrice.

Comme cela est connu en soi, le taux d'hématocrite, ou hématocrite, correspond au volume relatif occupé par les globules rouges par rapport au volume total de sang. Certains constructeurs d'instruments d'immunodosage qui utilisent, comme échantillon pour le diagnostic, un échantillon de sang total à la place d'un échantillon de plasma, ont proposé de corriger la dose d'analyte mesurée dans un échantillon de sang total selon la formule suivante :

$$D_p = \frac{D_{ST}}{1 - \frac{D_H}{100}} \qquad (1)$$

où $D_P$ est la dose d'analytes corrigée, $D_{ST}$ est la dose d'analytes mesurée directement sur un échantillon de sang total, $D_H$ est une mesure du taux d'hématocrite de l'échantillon de sang total. On peut noter que la relation (1) peut également s'appliquer avec les concentrations d'analytes mesurée et corrigée en lieu et place des doses correspondantes. Mais cette correction, qui s'apparente à une simple règle de trois, ne donne pas de bons résultats.

A ce jour, la dose d'un analyte, et corolairement sa concentration, est donc majoritairement déterminée sur le plasma, seule cette mesure étant considérée comme fiable et reproductible. En outre, cette mesure a l'avantage d'être indépendante du taux d'hématocrite, qui est variable d'un sujet à l'autre.

Or, l'obtention de plasma nécessite une étape préliminaire de centrifugation, et donc du temps et un appareillage spécifique. Non seulement, le temps peut être une donnée cruciale dans le cadre de diagnostics médicaux, notamment lorsqu'un pronostic vital est engagé, mais en outre cela suppose une grande quantité de centrifugeuses dans un laboratoire lorsque ce dernier est en charge d'une grande quantité d'analyses.

## EXPOSE DE L'INVENTION

Le but de la présente invention est de proposer un procédé de mesure de la dose, et donc de la concentration, d'un analyte directement sur un échantillon de sang total ayant un niveau de précision suffisant, permettant ainsi, notamment, d'éviter l'étape préliminaire de centrifugation, tout en délivrant une mesure indépendante du taux d'hématocrite.

A cet effet, l'invention a pour objet un procédé de mesure d'une dose plasmatique d'un analyte dans un échantillon de sang total, qui comporte :

- la mesure du taux d'hématocrite de l'échantillon de sang total;
- la mesure d'une dose d'analyte directement dans l'échantillon de sang total; et
- le calcul de la dose plasmatique d'analyte selon la relation :

$$D_P = P_a(D_{ST}, D_H)$$

où $D_P$ est la dose plasmatique d'analyte, $D_{ST}$ est la dose d'analyte mesurée, $D_H$ est le taux d'hématocrite mesuré, et $P_a$ est un polynôme non constant de degré supérieur ou égal à 1 dont les grandeurs indéterminées sont la dose d'analyte mesurée $D_{ST}$ et le taux mesuré d'hématocrite $D_H$, et dont les coefficients polynomiaux dépendent de l'analyte.

Une fois la dose d'analyte mesurée, cette dernière est usuellement traduite, par exemple directement par l'instrument de mesure, en concentration en analyte présent dans l'échantillon, car le volume de l'échantillon est fixe et connu. Comme c'est la valeur de concentration qui est utilisée, notamment pour mettre en oeuvre un diagnostic *in vitro,* en comparant la concentration mesurée à des valeurs de concentration de référence, prédéfinies, caractéristiques du diagnostic, l'invention a également pour objet un procédé de mesure d'une concentration plasmatique en analyte dans un échantillon de sang total, qui comporte :

- la mesure du taux d'hématocrite de l'échantillon de sang total;
- la mesure d'une dose d'analyte directement dans l'échantillon de sang total et sa transformation en concentration à partir du volume d'échantillon testé; et
- le calcul de la concentration plasmatique en analyte selon la relation :

$$C_P = P_a(C_{ST}, D_H)$$

où $C_P$ est la concentration plasmatique en analyte, $C_{ST}$ est la concentration en analyte calculée à partir de la dose mesurée et du volume de l'échantillon, $D_H$ est le taux d'hématocrite mesuré, et $P_a$ est un polynôme non constant de degré supérieur ou égal à 1 dont les grandeurs indéterminées sont la concentration en analyte obtenue à partir de la dose mesurée $C_{ST}$ et le taux mesuré d'hématocrite $D_H$, et dont les coefficients polynomiaux dépendent de l'analyte.

En d'autres termes, les inventeurs ont observé qu'il est possible de corriger sensiblement l'erreur sur la dose/concentration mesurée directement sur un échantillon de sang total à l'aide d'un polynôme non constant, notamment d'ordre supérieur ou égal à 1 dont les inconnues, indéterminées, ou variables, sont le taux d'hématocrite et la dose/concentration mesurée sur l'échantillon de sang total. La correction selon l'invention permet notamment d'obtenir une erreur relative de mesure par rapport à une mesure classiquement réalisée directement sur un échantillon de plasma contenue et souvent comprise dans la fourchette +/- 10% sur une gamme étendue de doses/concentrations. La dose/concentration corrigée, dont la valeur est suffisamment proche de celle qui aurait été obtenue en réalisant la mesure directement sur un échantillon de plasma, est ainsi exploitable, par exemple pour réaliser un diagnostic *in vitro,* de sorte qu'il n'est plus besoin de faire appel à une étape préliminaire de centrifugation.

Selon un mode de réalisation, le polynôme $P_a$ comprend le produit $D_{ST} \times D_H$ de la dose mesurée d'analyte $D_{ST}$ par le taux mesuré d'hématocrite $D_H$, ou bien $C_{ST} \times D_H$ de la concentration mesurée d'analyte $C_{ST}$ par le taux mesuré d'hématocrite $D_H$.

Plus particulièrement, le calcul de la dose plasmatique d'analyte est réalisé selon la relation :

$$D_P = a_0 + a_1 \times D_{ST} + a_2 \times D_H + a_{12} \times D_{ST} \times D_H$$

où $a_0$, $a_1$, $a_2$ et $a_{12}$ sont des coefficients prédéterminés dépendants de l'analyte.

En d'autres termes, le polynôme faisant intervenir l'interaction entre les termes $D_{ST}$ et $D_H$, à savoir les termes du premier ordre $D_{ST} \times D_H$, est particulièrement bien adapté aux techniques d'immunodosage de type ELISA, ELFA et immuno-capture.

Bien entendu, les coefficients $a_0$, $a_1$, $a_2$ et $a_{12}$ sont également dépendants du système de mesure (analyte et hématocrite).

De même, le calcul de la concentration plasmatique en analyte est réalisé selon la relation :

$$C_P = a'_0 + a_1 \times C_{ST} + a'_2 \times D_H + a_{12} \times C_{ST} \times D_H$$

où $a'_0$, $a_1$, $a'_2$ et $a_{12}$ sont des coefficients prédéterminés dépendants de l'analyte.

En d'autres termes, le polynôme faisant intervenir l'interaction entre les termes $C_{ST}$ et $D_H$, à savoir les termes du premier ordre $C_{ST} \times D_H$, est particulièrement bien adapté aux techniques d'immunodosage de type ELISA, ELFA et immuno-capture.

Bien entendu, les coefficients $a'_0$, $a_1$, $a'_2$ et $a_{12}$ sont également dépendants du système de mesure (analyte et hématocrite).

Notamment, lorsque l'analyte est un D-dimère dans une plage de mesure de concentration de 45 ng/ml à 1000 ng/ml, les coefficients sont donnés par les relations suivantes:

- $a'_0$ est compris entre -29,311 × 0,9 et -29,311 × 1,1;
- $a_1$ est compris entre 0,788 × 0,9 et 0,788 × 1,1;
- $a'_2$ est compris entre 0,702 × 0,9 et 0,702 × 1,1; et
- $a_{12}$ est compris entre 0,018 × 0,9 et 0,018 × 1,1.

Plus particulièrement :

- $a'_0$ = -29,311 ;
- $a_1$ = 0,788 ;
- $a'_2$ = 0,702 ; et
- $a_{12}$ = 0,018.

Notamment, lorsque l'analyte est une troponine, par exemple la tropine I cardiaque, dans une plage de concentration de mesure de 0,01 μg/l à 1,6 μg/l, les coefficients sont donnés par les relations suivantes:

- $a'_0$ est compris entre -0,0052 × 0,9 et -0,0052 × 1,1;
- $a_1$ est compris entre 0,9155 × 0,9 et 0,9155 × 1,1;
- $a'_2$ est compris entre 0,0002 × 0,9 et 0,0002 × 1,1; et
- $a_{12}$ est compris entre 0,0072 × 0,9 et 0,0072 × 1,1.

Plus particulièrement :

- $a'_0$ = -0,0052 ;
- $a_1$ = 0,9155 ;
- $a'_2$ = 0,0002 ; et
- $a_3$ = 0,0072.

Selon un mode de réalisation, la mesure de la dose et donc de la concentration de l'analyte est réalisée selon une technique de type ELISA ou de type ELFA ou de type immuno-capture. En d'autres termes, la technique de mesure utilisée n'est pas modifiée par rapport aux techniques de mesure réalisée directement sur du plasma. Les instruments d'immunodosage de l'état de la technique peuvent ainsi être utilisés.

L'invention a également pour objet un dispositif pour la mesure de la dose plasmatique d'un analyte dans un échantillon de sang total, *caractérisé* en ce qu'il comporte

- des moyens de réception dudit échantillon de sang total ;
- des moyens de mesure de la dose d'analyte réalisée sur l'échantillon de sang total ;
- des moyens de calcul de la dose plasmatique d'analyte selon la relation :

$$D_P = P_a(D_{ST}, D_H)$$

où $D_P$ est la dose plasmatique d'analyte, $D_{ST}$ est la dose d'analyte mesurée, $D_H$ est le taux d'hématocrite mesuré, et $P_a$ est un polynôme non constant de degré supérieur ou égal à 1 dont les grandeurs indéterminées sont la dose d'analyte mesurée $D_{ST}$ et le taux mesuré d'hématocrite $D_H$, et dont les coefficients polynomiaux dépendent de l'analyte.

L'invention a également pour objet un dispositif pour la mesure de la concentration plasmatique en analyte dans un échantillon de sang total, qui comporte :

- des moyens de réception dudit échantillon de sang total ;
- des moyens de mesure de la dose d'analyte réalisée sur l'échantillon de sang total ;
- des moyens pour transformer la dose d'analyte en concentration ;
- des moyens pour entrer ou pour mesurer le taux d'hématocrite dans l'échantillon de sang total ; et
- des moyens de calcul de la concentration plasmatique en analyte selon la relation :

$$C_P = P_a(C_{ST}, D_H)$$

où $C_P$ est la concentration plasmatique en anlyte, $C_{ST}$ est la concentration en analyte obtenue à partir de la dose mesurée, $D_H$ est le taux d'hématocrite mesuré, et $P_a$ est un polynôme non constant de degré supérieur ou égal à 1 dont les grandeurs indéterminées sont la concentration en analyte mesurée $C_{ST}$ et le taux mesuré d'hématocrite $D_H$, et dont les coefficients polynomiaux dépendent de l'analyte.

## BREVE DESCRIPTION DES FIGURES

L'invention sera mieux comprise à la lecture de la description qui va suivre, faite uniquement à titre d'exemple, et en relation avec les dessins annexés, dans lesquels :

- les figures 1A à 1G sont des vues schématiques illustrant une mesure de concentration d'analyte de type ELISA ou ELFA « sandwich » de l'état de la technique ;

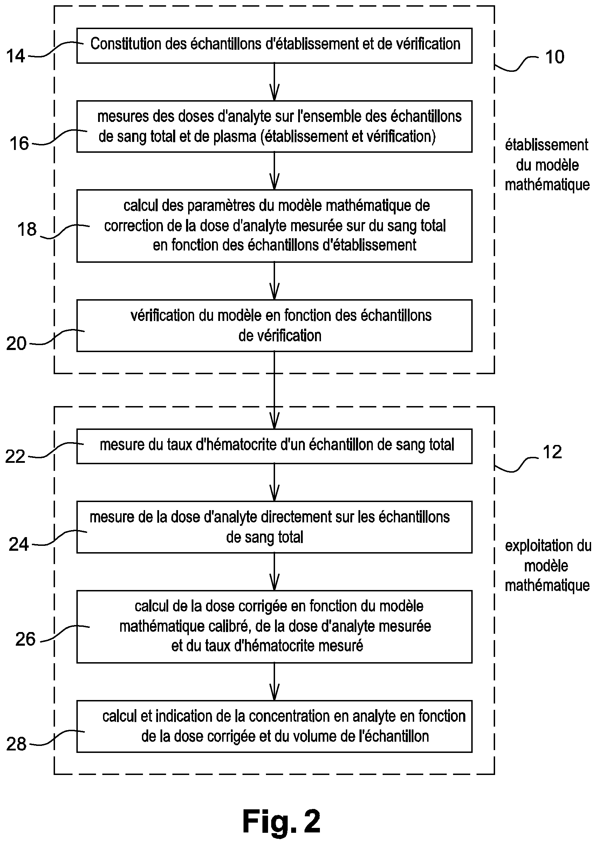

■ la figure 2 est un organigramme illustrant un procédé selon l'invention ;

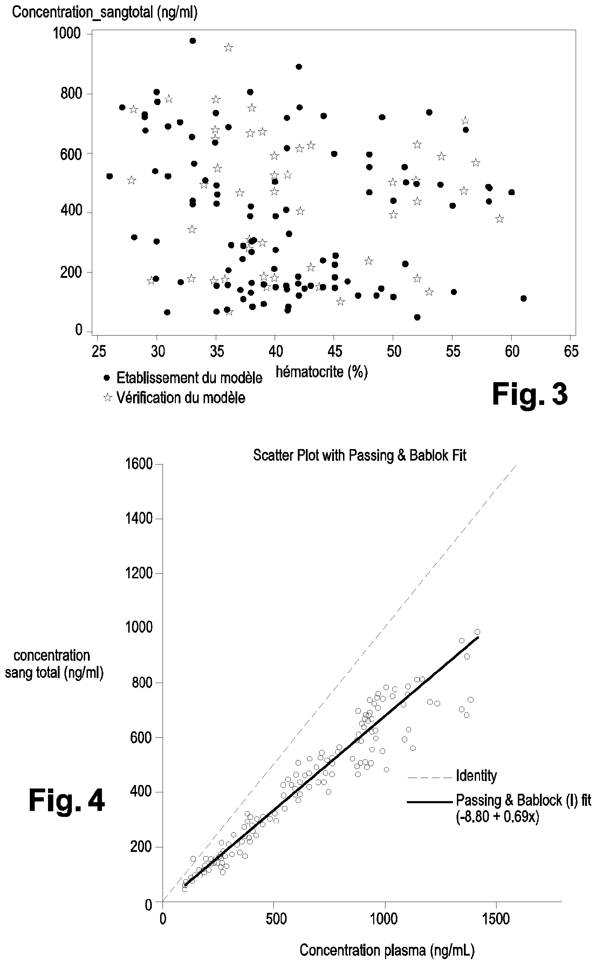

■ la figure 3 est un tracé illustrant l'homogénéité en concentration et en taux d'hématocrite d'échantillons utilisés pour l'établissement et la vérification d'un modèle polynômial de correction appliquée à des D-dimères ;

■ la figure 4 est un tracé illustrant la concentration en D-dimère mesurée dans du sang total, sans correction selon l'invention, en fonction de la concentration en D-dimère mesurée du plasma ;

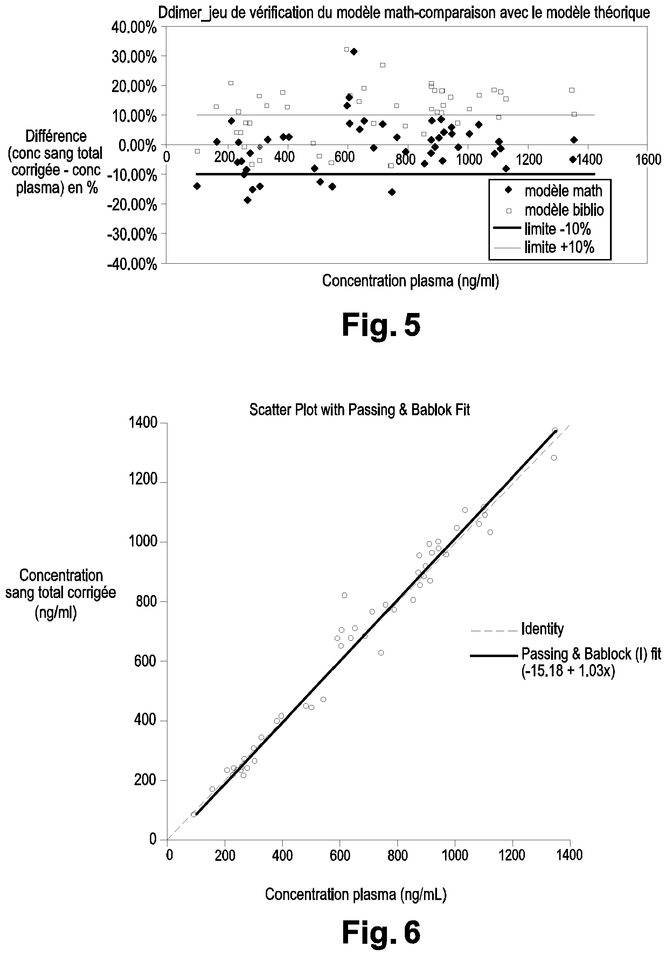

■ la figure 5 est un tracé, pour les échantillons du jeu de vérification, de l'erreur relative de correction obtenue selon l'invention et selon une correction de l'état de la technique, en fonction de la concentration en D-dimère mesurée dans du plasma ;

■ la figure 6 est un tracé, pour les échantillons du jeu de vérification, de la concentration en D-dimère corrigée selon l'invention en fonction de la concentration en D-dimère mesurée dans du plasma ;

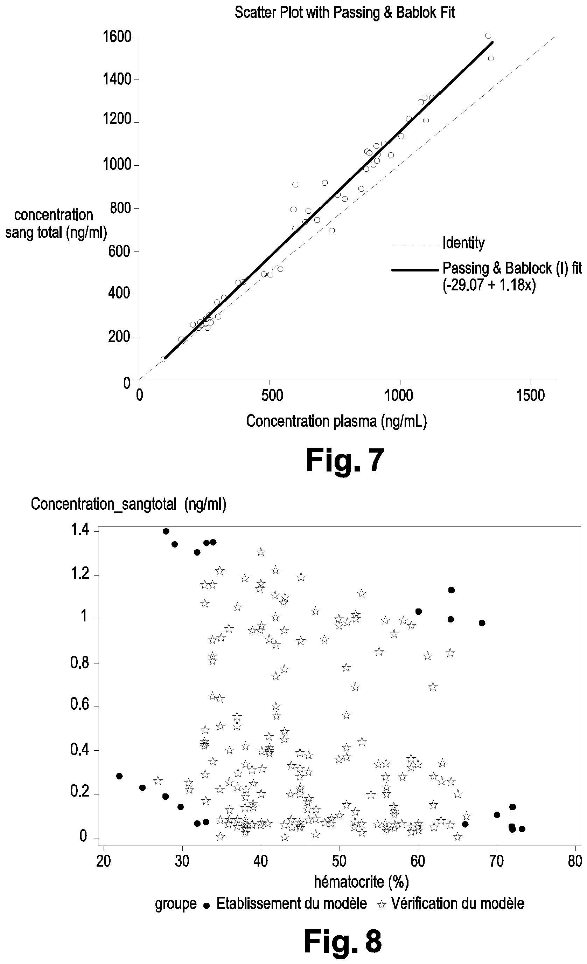

■ la figure 7 est un tracé de la concentration en D-dimère corrigée selon l'état de la technique en fonction de la concentration en D-dimère mesurée dans du plasma ;

■ la figure 8 est un tracé, pour les échantillons des jeux d'établissement et de vérification, illustrant l'homogénéité en concentration et en taux d'hématocrite d'échantillons utilisés pour l'établissement d'un modèle polynômial de correction appliquée dans le cadre de la quantification de la Troponine I cardiaque (TnI) ;

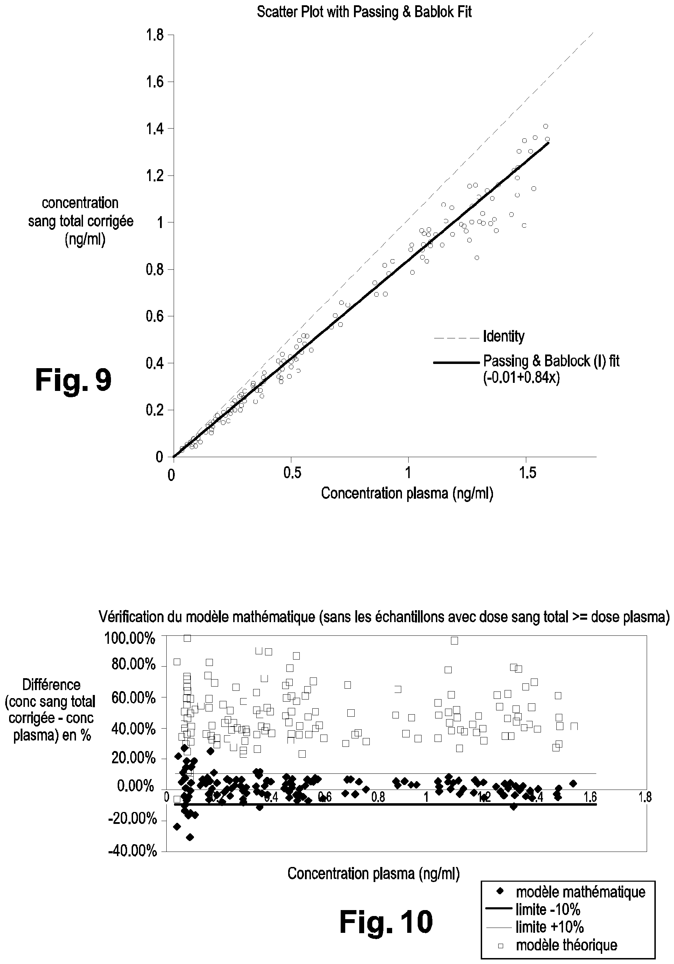

■ la figure 9 est un tracé illustrant la concentration en TnI mesurée dans des échantillons de sang total, sans correction selon l'invention, en fonction de la concentration en TnI mesurée dans des échantillons de plasma ;

■ la figure 10 est un tracé, pour les échantillons du jeu de vérification, de l'erreur relative de correction obtenue selon l'invention et selon une correction de l'état de la technique en fonction de la concentration en TnI mesurée dans du plasma ;

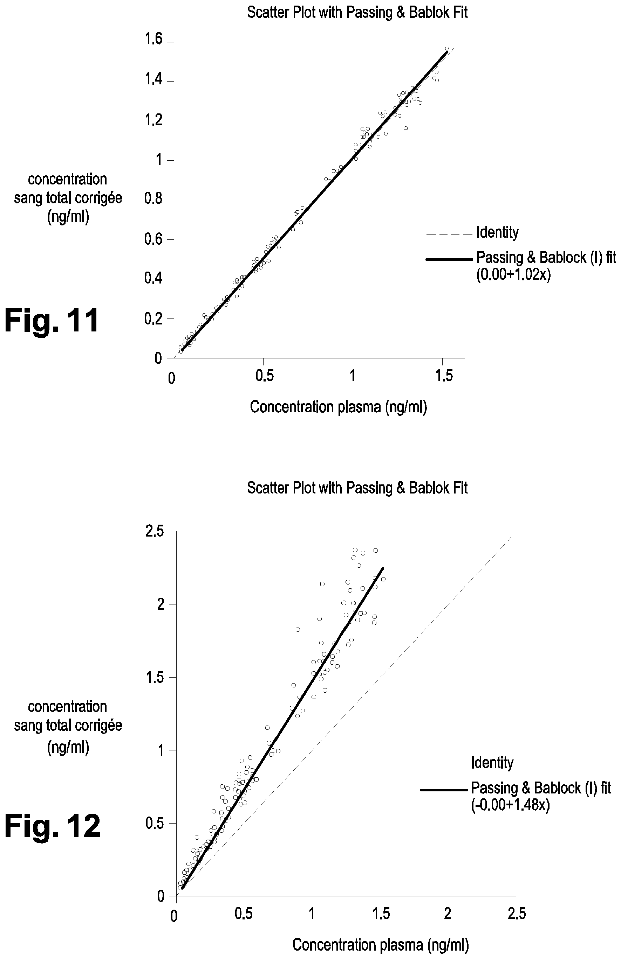

■ la figure 11 est un tracé de la concentration en TnI corrigée selon l'invention en fonction de la concentration en TnI mesurée dans du plasma ; et

■ la figure 12 est un tracé de la concentration en TnI corrigée selon l'état de la technique en fonction de la concentration en TnI mesurée dans du plasma.

## DESCRIPTION DETAILLEE DE L'INVENTION

En se référant à l'organigramme de la figure 2, il va à présent être décrit un procédé selon l'invention appliqué à un analyte particulier.

Le procédé comprend une étape **10** d'établissement d'un modèle mathématique corrigeant la mesure de la dose/concentration de l'analyte sur un échantillon de sang total, et une étape d'exploitation **12** du modèle mathématique établi à l'étape **10** afin de déduire une dose/concentration plasmatique inconnue en analyte dans un échantillon de sang total prélevé sur un patient ou un animal.

L'établissement du modèle **10** débute, en **14,** par la constitution d'un ensemble de paires d'échantillons de sang total et de plasma issues du même lot, par exemple du même patient ou animal, avec un taux variable d'hématocrite et une concentration variable d'analyte. Ces échantillons serviront à l'établissement et la vérification du modèle mathématique. De manière avantageuse, la variation d'hématocrite utilisée pour ces échantillons est supérieure à la gamme constatée pour l'homme ou l'animal dont est issu le sang, et de préférence centrée sur la moyenne constatée d'hématocrite chez le sujet ou une valeur proche de celle-ci. Par exemple, les valeurs normales de l'hématocrite chez l'homme sont de 40 à 54 %, avec une moyenne à 45%, et de 37 à 47 %, avec une moyenne à 42 % chez la femme, et la gamme de taux d'hématocrite utilisée pour l'établissement et la vérification du modèle mathématique est comprise entre 26% et 68%. La concentration en analyte dans le sang total est quant à elle choisie pour varier entre une valeur faible caractéristique d'un sujet sain et une valeur haute préparée par surcharge avec un maximum de 10% en volume de l'échantillon de sang total.

Par exemple, un ensemble de volumes de sang total est constitué, et chaque volume est divisé en deux, le premier sous-volume constituant l'échantillon de sang total et le second sous-volume étant centrifugé pour obtenir l'échantillon de plasma. Un volume de sang total peut provenir d'un seul sujet, provenir d'un mélange de plusieurs sang totaux prélevés sur différents sujets, être surchargé en analyte pour fixer la concentration en analyte, et/ou être issu d'un premier volume auquel une partie du plasma a été ôtée par centrifugation ou auquel du plasma issu d'une centrifugation a été rajouté pour fixer le taux d'hématocrite.

Le procédé se poursuit alors, en **16,** pour chaque paire d'échantillons, par la mesure de la dose ou concentration d'analyte et du taux d'hématocrite dans l'échantillon de sang total, et par la mesure de la dose ou concentration en analyte dans l'échantillon de plasma correspondant. La mesure des doses est par exemple réalisée au moyen d'une technique de type ELISA, ELFA ou d'immuno-capture de l'état de la technique. Il est ainsi obtenu un ensemble de triplets $(D_{ST}(i), D_P(i), D_H(i))$, chacun comprenant une dose $D_{ST}(i)$ d'analyte dans le sang total, une dose $D_P(i)$ d'analyte dans le plasma et un taux hématocrite $D_H(i)$ dans le sang total. Bien entendu, ceci est tout à fait applicable à la mesure de la concentration en remplaçant les doses d'analyte par les concentrations en analyte.

De manière avantageuse, les triplets présentant des valeurs aberrantes sont ensuite écartés, notamment ceux dont la dose $D_{ST}(i)$ mesurée dans le sang total est supérieure à la dose $D_P(i)$ mesurée dans le plasma. Là encore, ceci est applicable à la mesure de la concentration.

L'ensemble de paires d'échantillons obtenu est ensuite divisé en deux sous-ensembles, égaux ou non en nombre, un premier sous-ensemble étant utilisé pour l'établissement du modèle mathématique et un second sous-ensemble étant utilisé pour la vérification du modèle mathématique établi. L'ensemble des échantillons utilisés pour l'établissement du modèle mathématique sont notés $\left(D_{ST}^{cal}(i), D_P^{cal}(i), D_H^{cal}(i)\right)$ et l'ensemble des échantillons utilisés pour la vérification du modèle mathématique sont notés $\left(D_{ST}^{verif}(i), D_P^{verif}(i), D_H^{verif}(i)\right)$. Par exemple, deux tiers des paires d'échantillons sont utilisés pour l'établissement du modèle mathématique et un tiers des paires est utilisé pour la vérification de celui-ci.

Comme cela est connu en soi, la technique de mesure employée dépend de l'analyte en cause, notamment en raison des partenaires de liaison spécifiques utilisés pour fixer l'analyte, par exemple par immobilisation à la surface d'une surface solide, notamment un cône.

La mesure de la dose et de la concentration est mise en oeuvre au moyen d'un ou plusieurs immuno-analyseurs, comme par exemple l'automate VIDAS® de la société BioMérieux. Comme cela est connu en soi, un immuno-analyseur comprend une ou plusieurs sections de test apte(s) à recevoir chacune une ou plusieurs barrettes de test. Chaque barrette comprend plusieurs puits, un puits recevant l'échantillon à analyser, et les autres puits contenant respectivement les réactifs utilisés lors de la mesure, notamment du diluant, une solution de rinçage, une solution comprenant le conjugué et une solution contenant le substrat enzymatique. L'automate comprend en outre un mécanisme de déplacement de la barrette en dessous d'un cône dont la surface contient une couche de partenaires de liaison spécifiques à l'analyte. Le cône est alors positionné au-dessus de chaque puits et pipette dans un ordre précis les différents milieux présents dans ceux-ci tout en mettant en oeuvre des mécanismes d'aspiration, de refoulement et d'incubation propre à la technique de mesure employée. L'automate comprend enfin un dispositif pour la mesure de la propriété impliquée dans cette dernière. Par exemple, l'automate VIDAS® applique une technique de type ELFA, qui diffère de la technique ELISA directe décrite précédemment en ce que le substrat catalysé par la fonction enzymatique produit de la fluorescence, et il comporte un fluorimètre permettant de mesurer la fluorescence de la solution contenue dans la dernière cuvette une fois la dernière étape de lavage effectuée.

La mesure du taux d'hématocrite est quant à elle réalisée à l'aide de toute technique connue appropriée, par exemple à l'aide de la technique dite de microhématocrite utilisée dans le présent mode de réalisation, de Coulter, par mesure laser, ou par mesure de conductivité.

Dans une étape **18** suivante du procédé, un calcul est mis en oeuvre en fonction des triplets d'établissement, par exemple $\left(D_{ST}^{cal}(i), D_P^{cal}(i), D_H^{cal}(i)\right)$, pour calculer les paramètres d'un modèle mathématique de correction d'une dose d'analyte mesurée directement sur un échantillon de sang total, plus particulièrement un modèle selon la relation :

$$D_P = a_0 + a_1 \times D_{ST} + a_2 \times D_H + a_{12} \times D_{ST} \times D_H \qquad (2)$$

où $D_P$ est la dose plasmatique d'analyte, $D_{ST}$ est la dose d'analyte mesurée directement sur un échantillon de sang total, $D_H$ est le taux d'hématocrite de l'échantillon de sang total, et $a_0$, $a_1$, $a_2$ et $a_{12}$ sont les coefficients calculés du modèle mathématique.

De manière avantageuse, avant d'appliquer l'algorithme de calculs des coefficients polynomiaux, les variables $D_{ST}$ et $D_H$ du polynôme selon la relation (2) sont normalisées entre -1 et 1. Notamment, si la dose d'analyte dans le sang total a pour valeur minimale $D_{ST}^{min}$ et a pour valeur maximale $D_{ST}^{max}$, la variable $D_{ST}$ est transformée en la variable $X_{ST} = \frac{D_{ST}-c}{d}$, où $c = \frac{D_{ST}^{max}+D_{ST}^{min}}{2}$ et $d = \frac{D_{ST}^{max}-D_{ST}^{min}}{2}$. De même, si le taux d'hématocrite a pour valeur minimale $D_H^{min}$ et a pour valeur maximale $D_H^{max}$, la variable $D_H$ est transformée en la variable $X_H = \frac{D_H-e}{f}$, avec $e = \frac{D_H^{max}+D_H^{min}}{2}$ et $f = \frac{D_H^{max}-D_H^{min}}{2}$.

Le modèle selon la relation (2) se réécrit alors selon la relation :

$$D_p = a_0^n + a_1^n \times X_{ST} + a_2^n \times X_H + a_{12}^n \times X_{ST} \times X_H \qquad (3)$$

où les coefficients $a_0^n$, $a_1^n$, $a_2^n$ et $a_{12}^n$ sont déterminés selon la méthode des moindres carrés en minimisant une fonction de coût $f(D_P^{cal}(i) - (a_0^n + a_1^n \times X_{ST}^{cal}(i) + a_2^n \times X_H^{cal}(i) + a_{12}^n \times X_{ST}^{cal}(i) \times X_H^{cal}(i))$ Les coefficients $a_0$, $a_1$, $a_2$ et $a_{12}$ se déduisent aisément des coefficients $a_0^n$, $a_1^n$, $a_2^n$ et $a_{12}^n$

La transformation des variables est indépendante de l'algorithme de calcul et permet d'exprimer chaque variable dans la même échelle et pouvoir ainsi comparer les différents coefficients $a_1^n$, $a_2^n$ et $a_{12}^n$.

Là encore, ce qui précède est tout à fait applicable à la mesure de concentration en remplaçant la dose d'analyte en concentration d'analyte. Ainsi, en variante, la dose d'analyte mesurée dans l'échantillon de sang total est transformée en concentration d'analyte en divisant la dose mesurée par le volume de l'échantillon de sang total, puis un modèle mathématique de correction de la concentration est calculé, notamment un modèle selon la relation :

$$C_P = a'_0 + a_1 \times C_{ST} + a'_2 \times D_H + a_{12} \times C_{ST} \times D_H \qquad (2bis)$$

où $C_P$ est la concentration plasmatique en analyte, $C_{ST}$ est la concentration en analyte calculée à partir de la dose mesurée et du volume de l'échantillon, et où $a'_0$, $a_1$, $a'_2$ et $a_{12}$ sont des coefficients prédéterminés dépendants de l'analyte, ces coefficients étant calculés par exemple par une méthode des moindres carrés. On notera que, puisque la concentration en analyte est déduite de la dose, les coefficients liés à la concentration en analyte dans le polynôme, à savoir coefficients $a_1$ et $a_{12}$ sont les mêmes pour les deux modèles exprimés en dose et en concentration. En revanche, les autres coefficients, à savoir $a'_0$, et $a'_2$, sont différents.

De même, il est possible d'appliquer une normalisation des variables du polynôme de la relation (2bis) analogue à la normalisation de la relation (3), en calculant un modèle mathématique selon la relation :

$$C_p = a_0^{n'} + a_1^{n'} \times Y_{ST} + a_2^{n'} \times X_H + a_{12}^{n'} \times Y_{ST} \times X_H \qquad (3bis)$$

où $Y_{ST} = \frac{C_{ST} - g}{h}$, $g = \frac{C_{ST}^{max} + C_{ST}^{min}}{2}$, $h = \frac{C_{ST}^{max} - C_{ST}^{min}}{2}$, $C_{ST}^{min}$ est la valeur minimum de la concentration $C_{ST}$, $C_{ST}^{max}$ est la valeur maximale de la concentration $C_{ST}$, et les coefficients polynomiaux sont des coefficients se déduisant aisément des coefficients polynomiaux de la relation (2bis).

Le procédé se poursuit alors, en **20,** par la vérification du modèle établi en utilisant l'ensemble de triplets de vérification, par exemple $\left( D_{ST}^{verif}(i), D_P^{verif}(i), D_H^{verif}(i) \right)$ pour le modèle en dose.

Plus particulièrement, le modèle établi est avantageusement vérifié sur la base d'au moins un des critères suivants :

- l'erreur relative $\frac{D_P^{cor}(i) - D_P^{verif}(i)}{D_P^{verif}(i)}$ entre la dose corrigée $D_P^{cor}(i)$ par le modèle mathématique, à savoir $D_P^{cor}(i) = a_0 + a_1 \times D_{ST}^{verif}(i) + a_2 \times D_H^{verif}(i) + a_{12} \times D_{ST}^{verif}(i) \times D_H^{verif}(i)$, et la dose $D_P^{verif}$ mesurée dans le plasma. Plus particulièrement, il est vérifié si l'erreur relative est centrée sur 0% et est comprise entre -10% et +10% ;

- les paramètres d'une équation linéaire, par exemple obtenue selon une régression linéaire dite de « *Passing and Bablok* », $D_P^{cor}(i) = \alpha + \beta \times D_P^{verif}(i)$; et

- des biais calculés à différentes doses d'analyte dans le plasma en utilisant l'équation de « *Passing and Bablok* » décrite ci-dessus

Là encore, ce qui précède est tout à fait applicable à la mesure de concentration en remplaçant la dose d'analyte en concentration en analyte.

Une fois, le modèle établi validé, le procédé se poursuit par l'exploitation, en **12,** de ce modèle pour la mesure de dose/concentration d'analyte directement réalisée sur des échantillons de sang total.

Par exemple, le modèle est embarqué dans l'unité de traitement de données d'un immuno-analyseur du commerce, l'immo-analyseur pouvant en outre être modifié pour pouvoir recevoir ou calculer des valeurs de taux d'hématocrite. Ou bien, le modèle est mis en oeuvre sur une unité de traitement indépendante de l'immuno-analyseur, par exemple un ordinateur personnel. Dans cette variante, un opérateur entre dans l'unité la valeur de dose/concentration d'analyte mesurée par l'immuno-analyseur sur un échantillon de sang total et la valeur du taux d'hématocrite mesuré pour cet échantillon et obtient en retour la dose/concentration d'analyte corrigée.

Ainsi, pour un échantillon de sang total de volume connu dont on cherche la dose, mais surtout la concentration d'analyte, l'étape d'exploitation **12** débute, en **22,** par la mesure du taux d'hématocrite $D_H$ de l'échantillon, et se poursuit, en **24,** par la mesure de la dose/concentration d'analyte directement sur l'échantillon de sang total au moyen de la même technique de mesure, mise en oeuvre par un même modèle d'immuno-analyseur, que celle utilisée pour l'établissement et la vérification du modèle mathématique.

Une dose d'analyte corrigée $D_P$ est alors calculée, en **26,** selon la relation (2) avec les coefficients $a_0$, $a_1$, $a_2$ et $a_{12}$ calculés lors de l'établissement du modèle **10,** ou de manière équivalente les coefficients $a_0^n$, $a_1^n$, $a_2^n$ et $a_{12}^n$ du modèle selon la relation (3). Une concentration en analyte corrigée est calculée de la même manière selon la relation (2bis) ou (3bis).

En **28,** la concentration est calculée puis délivrée, par exemple affichée sur un écran et/ou enregistrée dans une mémoire informatique. Optionnellement, la dose corrigée peut également être délivrée.

Il a été décrit un procédé selon l'invention dans lequel la dose ou la concentration mesurée est corrigée par un modèle mathématique.

Comme décrit précédemment, les techniques de mesure se basent sur la mesure d'une propriété, par exemple optique, électrique, chimique, de pH, ou enzymatique, dont la valeur dépend de la dose d'analytes présents dans l'échantillon analysé. Un immuno-analyseur comporte ainsi un dispositif qui mesure une telle propriété et délivre un signal correspondant à cette mesure. Selon l'état de la technique, le signal est ensuite traité à l'aide d'un modèle mathématique prédéterminé qui transforme le signal en une valeur de dose et/ou de concentration.

En variante, le procédé s'applique au signal lui-même, avant sa transformation. Notamment, le signal mesuré sur l'échantillon de sang total est corrigé selon la relation :

$$S_P = a_0 + a_1 \times S_{ST} + a_2 \times D_H + a_{12} \times S_{ST} \times D_H \quad (4)$$

où $S_P$ est le signal corrigé et $S_{ST}$ est le signal issu de la mesure sur l'échantillon de sang total. Le procédé appliqué à la correction du signal est analogue au procédé décrit précédemment sous couvert de modifications mineures à la portée de l'homme du métier. Le calcul d'un modèle mathématique avec des variables normées analogues à la relation (3) est également possible.

Il va à présent être décrit deux exemples d'application de l'invention, respectivement une application à la mesure d'une concentration en D-dimère et une application à la mesure d'une concentration en troponine I cardiaque.

Mesure de la concentration en D-dimère

Les D-dimères sont des produits hétérogènes de la fibrine possédant tous l'épitope D-D formée par deux monomères de fibrine contigus liés de manière covalente par l'enzyme responsable de la coagulation sanguine ramifiant la fibrine, ou facteur « XIIIa ». Le dosage des D-dimères est indiqué principalement dans le diagnostic d'exclusion des maladies thromboemboliques veineuses, notamment la thrombose veineuse profonde et l'embolie pulmonaire, l'évaluation du risque de récidive de telles maladies après l'arrêt d'un traitement anticoagulant, et dans le diagnostic de coagulation intravasculaire disséminée.

Pour l'établissement et la vérification du modèle mathématique associé au D-dimère, du sang a été prélevé chez 186 sujets sains sur du citrate de sodium en tant qu'anticoagulant. Plus particulièrement, le sang a été collecté dans des tubes de 4,5 mL sous vide contenant 0,129 M de citrate de trisodium.

Afin d'avoir des échantillons de sang total présentant un taux d'hématocrite varié, les échantillons collectés ont été dilués avec leur propre plasma après une faible centrifugation, de manière à obtenir un taux d'hématocrite variant dans la gamme de 26% à 61 %. Le taux d'hématocrite est mesuré au moyen de la technique de microhématocrite. La microhématocrite est tout d'abord acquise par centrifugation de capillaires pendant 7 minutes à 10 000 tr/min puis la valeur du taux d'hématocrite est ensuite déduite à l'aide d'un abaque, comme cela est connu en soi de l'état de la technique.

Afin d'avoir des échantillons de sang total et de plasma présentant une concentration en D-dimère variée, les échantillons de sang total ont été surchargés en D-dimère, avant ou après avoir fait varier le taux d'hématocrite. Pour surcharger les échantillons, des plasmas provenant de la sérothèque interne à la société bioMérieux (Marcy L'Etoile, France) et fortement concentrés en D-dimère à doser ont été utilisés afin d'avoir une répartition de la concentration en D-dimère dans le sang total répartie dans la gamme 45ng/mL-1000ng/mL. Les échantillons de sang total ont été surchargés notamment à 300 ng/mL, 500 ng/mL et 1000 ng/mL, la surcharge ne dépassant pas 10 % du volume de l'échantillon de sang total afin de ne pas modifier la matrice originale du sang, c'est-à-dire ne dépassant pas 1000 ng/mL. La répartition des échantillons est uniforme dans la gamme de la concentration en D-dimère obtenue à partir de la dose mesurée dans le sang total et dans la gamme du taux d'hématocrite, comme cela est illustré à la figure 3 qui représente les couples (concentrations issues des doses mesurées dans le sang total, taux d'hématocrite) pour les triplets d'établissement du modèle (ronds) et pour les triplets de vérification du modèle (étoiles). Les échantillons de plasma sont par ailleurs obtenus par centrifugation d'une partie de chaque échantillon de sang total à 3000 tr/min pendant 10 minutes.

A titre d'exemple numérique, suite à la préparation d'échantillons, la concentration en D-dimère dans le sang total est répartie dans la gamme 46,78 ng/mL-982,81 ng/mL, le taux d'hématocrite est réparti dans la gamme de 26% à 61%, et la concentration en D-dimère dans le plasma est répartie dans la gamme 96,86 ng/mL-1419,58 ng/mL.

La mesure de la concentration en D-dimère dans les échantillons de sang total et de plasma consiste en la détermination immunoenzymatique des produits de dégradation de la fibrine (PDF) dans le plasma humain par une technique de type ELFA, mise en oeuvre notamment par un automate VIDAS® avec le kit « VIDAS D-dimère Exclusion II » de référence 30 455 de la société bioMérieux SA. Cette mesure associe donc la méthode immunoenzymatique de type sandwich en deux étapes à une détection finale en fluorescence (ELFA). Dans une première étape, l'échantillon est prélevé, aspiré et refoulé plusieurs fois afin que l'antigène puisse se fixer aux anticorps anti-FbDP (FbDP pour « *fibrinogen degradation products* » - produit de dégradation de la fibrine) fixés sur le cône. Lors d'une deuxième étape, un anticorps monoclonal anti-FbDP marqué à la PAL (phosphatase alcaline) se lie à l'antigène déjà fixé sur le cône pour former un sandwich. Des étapes de lavage éliminent les composés non fixés ou en excès. L'étape de révélation est ensuite effectuée. Le substrat 4MUP (4-méthyl-umbelliféryl-phosphate) est aspiré puis refoulé dans le cône et la PAL catalyse l'hydrolyse du substrat en 4MU (4-méthyl-umbelliférone) fluorescent. La fluorescence émise est mesurée à 450 nm. La valeur du signal de fluorescence est proportionnelle à la dose de l'antigène dans l'échantillon.

La figure 4 illustre les concentrations en D-dimère obtenues à partir des concentrations $C_{ST}(i)$ mesurées dans les échantillons de sang total en fonction des concentrations en D-dimère obtenues à partir des doses $Cp(i)$ mesurées dans les échantillons de plasma respectifs. Une régression linéaire du type « *Passing and Bablok* » $C_{ST}(i) = \alpha + \beta \times C_P(i)$ entre les deux doses donne notamment $\alpha$ = -8,80 et $\beta$ = 0,69.

Les triplets pour lesquels les concentrations mesurées dans le sang total sont supérieures aux concentrations mesurées dans le plasma ont été écartés.

Cent trois triplets $\left( C_{ST}^{cal}(i), C_P^{cal}(i), C_H^{cal}(i) \right)$ ont été utilisés pour établir le modèle mathématique.

Il est ainsi obtenu les coefficients suivants :

- $a_0' = 806,279;$
- $a_1^n = 803,998$ avec un écart type égal à 11,917;
- $a_2' = 196,295$ avec un écart type égal à 12,389; et
- $a_{12}^n = 182,254$ avec un écart type égal à 25,909.

où de manière équivalente, sans normalisation :

- $a_0' = -29,311$ ;
- $a_1$ = 0,788 ;
- $a_2' = 0,702$ ; et
- $a_{12}$ = 0,018.

Cinquante triplets de vérification $\left( C_{ST}^{verif}(i), C_P^{verif}(i), C_H^{verif}(i) \right)$ ont par ailleurs été utilisés pour vérifier le modèle mathématique.

La figure 5 illustre l'erreur relative $\frac{C_P^{cor}(i) - C_P^{verif}(i)}{C_P^{verif}(i)}$ en fonction de la concentration en D-dimère obtenue à partir de la dose mesurée dans le plasma à la fois pour une correction $C_P^{cor}(i)$ selon la relation (2bis) ou (3bis) appliquée aux échantillons de vérification, représentée par des losanges, et pour une correction $C_P^{cor}(i)$ de l'état de la technique réalisée selon la relation (1) appliquée aux échantillons de vérification, représentée par les carrés. Comme on peut le noter, contrairement à la correction de l'état de la technique, l'erreur relative obtenue par l'invention est centrée sur zéro, et est comprise pour l'essentiel entre -10% et 10%.

Les figures 6 et 7 illustrent les concentrations corrigées en fonction des concentrations mesurées sur du plasma respectivement selon la correction de relation (2bis) ou (3bis) et selon la correction de la relation (1), et appliquées aux échantillons de vérification. La droite obtenue par régression linéaire du type « *Passing and Bablok* » pour chacune des corrections est en outre calculée et tracée. La droite correspondant à la correction de l'état de la technique a une pente égale à 1,18. La droite correspondant à l'invention est quant à elle proche de l'identité avec une pente égale à 1,03.

Enfin, les biais pour trois concentrations en D-dimère dans le sang total, à savoir 250 ng/mL, 500 ng/mL et 1000 ng/mL, ont été calculés et valent respectivement -3,4%, -0,4% et +1,1% pour la correction selon l'invention, et valent respectivement +6,7%, +12,5% et +15,4% pour la correction de l'état de la technique.

On observe des concentrations corrigées selon l'invention comparables à celles obtenues par une mesure réalisée directement sur du plasma.

On observe des résultats similaires lorsque la correction est réalisée sur le signal mesuré sur les échantillons.

Mesure de la concentration en troponine I cardiaque

Les troponines (Tn) sont des protéines du muscle trié. Le muscle strié est composé d' un filament épais, constitué de myosine, et d'un filament fin constitué d'actine, de tropomyosine et du complexe de troponine. Ce complexe est lui-même composé de trois sous-unités, à savoir les troponines dites « C », « I » et « T ». Chacune de ces troponines possède des isoformes squelettiques et cardiaques. Les troponines cardiaques sont des marqueurs de choix pour la détection de la nécrose myocardique et leur dosage permet de diagnostiquer un infarctus du myocarde. Le dosage des troponines cardiaques sert également pour le suivi d'un traitement thrombolytique et pour estimer la taille de la nécrose du myocarde, ainsi que dans le diagnostic de syndromes coronariens aigus. Le dosage de la troponine « I », notée TnI, permet également de mettre en évidence une atteinte cardiaque dans le cadre d'autres pathologies, notamment une insuffisance rénale, une hypothyroïdie, des collagénoses, des myopathies ou encore une embolie pulmonaire.

Pour l'établissement et la vérification du modèle mathématique associé à la TnI, du sang a été prélevé chez 186 sujets sains sur de l'héparinate de lithium en tant qu'anticoagulant. Plus particulièrement, le sang a été collecté dans des tubes de 4 mL contenant 17 UI/mL d'héparinate de lithium.

L'obtention d'échantillons de sang total avec des taux d'hématocrite et de concentration de TnI, et l'obtention d'échantillons de plasma à partir des échantillons de sang total sont analogues à celles décrites en relation avec le dosage des D-dimères.

La mesure de concentration en TnI dans les échantillons de sang total et de plasma consiste en la détermination immunoenzymatique de cette troponine dans le plasma humain par une technique de type ELFA, mise en oeuvre notamment par un automate VIDAS® avec le kit « VIDAS Troponine I Ultra » de référence 30 448 de la société bioMérieux. Au cours d'une seule étape, l'échantillon est prélevé et transféré dans le puits contenant les conjugués qui sont des anticorps anti-TNI cardiaque marqués à la PAL. Le mélange échantillon/conjugué est aspiré puis refoulé plusieurs fois par le cône. Cela permet à la TnI d'une part de se fixer sur les anticorps du cône et d'autre part de se lier au conjugué pour former un sandwich. Des étapes de lavage éliminent les composés non fixés ou en excès. La PAL catalyse l'hydrolyse du substrat contenu dans le dernier puits de la barrette VIDAS® en 4MU fluorescent. La fluorescence émise est mesurée à 450 nm. La valeur du signal de fluorescence est proportionnelle à la concentration de l'antigène dans l'échantillon. A la fin du test, les résultats sont calculés à partir de deux courbes de calibration correspondant aux deux étapes de révélation. Un signal seuil gère le choix de la courbe de calibration à utiliser pour chaque échantillon.

La concentration en TnI dans les échantillons de sang total varie de 0,01 µg/L à 1,4 µg/L et le taux d'hématocrite varie de 22% à 73%, la concentration en TnI dans le plasma variant ainsi de 0,01 µg/l à 1,6 µg/l.

La répartition des échantillons est uniforme dans la gamme de concentration en TnI mesurée dans le sang total et dans la gamme du taux d'hématocrite, comme cela est illustré à la figure 8 qui représente les couples (concentrations mesurées dans le sang total, taux d'hématocrite) pour les triplets d'établissement du modèle (ronds) et pour les triplets de vérification du modèle (étoiles).

La figure 9 illustre la concentration en TnI $C_{ST}(I)$ obtenue à partir des concentrations mesurées dans les échantillons de sang total en fonction de la concentration en TnI Cp(i) mesurée dans les échantillons de plasma respectifs.

Une régression linéaire du type « *Passing and Bablok* » entre les deux concentrations donne notamment $\alpha$ = -0,01 et $\beta$ = 0,84.

Cent quatre-vingt-sept échantillons ont été préparés au total et un algorithme d'échange, par exemple de Fedorov, est mis en oeuvre, par exemple par le logiciel NEMRODW© de la société LPRAI Sarl. L'algorithme de Fedorov est un algorithme itératif qui présente l'avantage de permettre de sélectionner les N meilleurs échantillons pour établir le modèle mathématique, par exemple les 20 meilleurs échantillons, parmi l'ensemble des échantillons pour calculer les paramètres du modèle mathématique.

A l'aide des vingt échantillons choisis, il est obtenu les coefficients suivants :

- $a'_0 = 0,8982;$
- $a^n_1 = 0,86751$ ;
- $a'_2 = 0,1334$ ; et
- $a^n_{12} = 0,12413.$

où de manière équivalente :

- $a'_0 = $ -0,0052 ;
- $a_1 = 0,9155$ ;
- $a'_2 = 0,0002$ ; et
- $a_{12} = 0,0072.$

Cent soixante-sept triplets de vérification $\left( C_{ST}^{verif}(i), C_{P}^{verif}(i), C_{H}^{verif}(i) \right)$ ont par ailleurs été utilisés pour vérifier le modèle mathématique.

La figure 10 illustre l'erreur relative $\dfrac{C_P^{cor}(i) - C_P^{verif}(i)}{C_P^{verif}(i)}$ en fonction de la concentration en TnI obtenue à partir de la dose mesurée dans le plasma à la fois pour une correction $C_P^{cor}(i)$ selon la relation (2bis) ou (3bis) appliquée aux échantillons de vérification, représentée par des losanges, et pour une correction $C_P^{cor}(i)$ de l'état de la technique réalisée selon la relation (1) appliquée aux échantillons de vérification, représentée par les carrés. Comme on peut le noter, contrairement à la correction de l'état de la technique, l'erreur relative est centrée sur zéro, et est comprise pour l'essentiel entre -10% et 10%. On observe quelques résultats en dehors de -/+10% dans la gamme d'échantillon où la concentration en TnI est proche de la limite de détection.

Les figures 11 et 12 illustrent les concentrations en TnI corrigées en fonction des concentrations mesurées sur du plasma respectivement selon la correction de relation (2bis) ou (3bis) et la correction de la relation (1), appliquées aux échantillons de vérification. La droite obtenue par régression linéaire du type « *Passing and Bablok* » pour chacune des corrections est en outre calculée et tracée. La droite correspondant à la correction de l'état de la technique a une pente égale à 1,48. La droite correspondant à l'invention est quant à elle proche de l'identité avec une pente égale à 1,02.

Enfin, les biais pour trois concentrations en TnI dans le sang total, à savoir 0,1μg/L, 0,5μg/L et 1μg/L, ont été calculés et valent respectivement 3,1%, 2,0% et +1,9% pour la correction selon l'invention, et valent respectivement +43,3%, +46,7% et +47,1 % pour la correction de l'état de la technique.

On observe également les mêmes résultats que ceux obtenus pour les D-dimères, à savoir des concentrations corrigées qui sont comparables à celles obtenues par une mesure réalisée directement sur du plasma.

On observe des résultats similaires lorsque la correction est réalisée sur le signal mesuré sur les échantillons.

Il a été décrit des modes de réalisation dans lesquels on utilise un polynôme du premier degré selon la relation (2bis) ou la relation (3bis). Un polynôme du second degré comprenant le carré du taux d'hématocrite et/ou le carré de la dose/concentration d'analyte mesurée dans le sang total peut également être utilisé. De même, un polynôme de degré plus élevé peut être utilisé.

Notamment, il peut être utilisé la procédure suivante pour identifier le polynôme utilisé :

a) fixer l'ordre du polynôme à 1 ;
b) calculer les paramètres du polynôme, par exemple au moyen d'une méthode des moindres carrés, tel que décrit précédemment ;
c) si l'erreur de prédiction du polynôme est jugée satisfaisante, retenir ce polynôme. Par exemple, l'erreur de prédiction est calculée et si celle-ci est inférieure à un seuil, avantageusement 10%, le polynôme est retenu ; et
d) si l'erreur de prédiction, avantageusement calculée sur un jeu de données de vérification, n'est pas jugée satisfaisante, augmenter l'ordre du polynôme d'une unité et réitérer les étapes b), c) et d) jusqu'à ce que l'erreur de prédication soit jugée satisfaisante. En variante, on réitère ces étapes tant que l'ordre est inférieur ou égal à 10, et de préférence inférieur ou égal à 5.

Bien entendu, d'autres méthodes de calcul et/ou de choix final du polynôme peuvent être utilisées. Par exemple, l'ordre peut être également calculé à l'aide d'une régression non linéaire, comme cela est connu en soi de l'état de la technique. De même, un critère de type bayésien, notamment le critère BIC (pour « bayesian information criterion ») peut être utilisé pour choisir le polynôme final lorsque plusieurs polynôme ont été calculés. Il a été décrit des modes de réalisation dans lequel la mesure de dose/concentration est utilisée sans transformation des variables dans les modèles mathématiques. En variante, une transformation de l'espace des mesures peut être mise en oeuvre, par exemple une transformation logarithmique si la gamme des doses/concentrations mesurées est importante. Le terme de « mesure » signifie donc au sens de l'invention la mesure directe ou transformée.

De même, il a été décrit des coefficients polynomiaux calculés pour une gamme importante de doses/concentrations. En variante, la gamme des doses/concentrations peut être divisée en une pluralité d'intervalles et un modèle mathématique être établi pour chacun de ces intervalles. Les coefficients du modèle peuvent ainsi dépendre de l'intervalle de doses/concentrations pour lequel ils sont calculés.

## Revendications

1. Procédé de mesure d'une dose plasmatique d'un analyte dans un échantillon de sang total, *caractérisé* **en ce qu'**il comporte :

   - la mesure du taux d'hématocrite de l'échantillon de sang total;
   - la mesure d'une dose d'analyte directement dans l'échantillon de sang total; et
   - le calcul de la dose plasmatique d'analyte selon la relation :

$$D_P = P_a(D_{ST}, D_H)$$

   où $D_P$ est la dose plasmatique d'analyte, $D_{ST}$ est la dose d'analyte mesurée, $D_H$ est le taux d'hématocrite mesuré, et $P_a$ est un polynôme non constant de degré supérieur ou égal à 1 dont les grandeurs indéterminées sont la dose d'analyte mesurée $D_{ST}$ et le taux mesuré d'hématocrite $D_H$, et dont les coefficients polynomiaux dépendent de l'analyte.

2. Procédé de mesure d'une concentration plasmatique en analyte dans un échantillon de sang total, *caractérisé* **en ce qu'**il comporte :

   - la mesure du taux d'hématocrite de l'échantillon de sang total;
   - la mesure d'une dose d'analyte directement dans l'échantillon de sang total et la transformation de la dose mesurée en une concentration en divisant la dose mesurée par le volume de l'échantillon ; et
   - le calcul de la concentration plasmatique en analyte selon la relation :

$$C_P = P_a(C_{ST}, D_H)$$

   où $C_P$ est la concentration plasmatique en analyte, en analyte corrigée, $C_{ST}$ est la dose d'analyte mesurée, $D_H$ est le taux d'hématocrite mesuré, et $P_a$ est un polynôme non constant de degré supérieur ou égal à 1 dont les grandeurs indéterminées sont la concentration en analyte mesurée $C_{ST}$ et le taux mesuré d'hématocrite $D_H$, et dont les coefficients polynomiaux dépendent de l'analyte.

3. Procédé selon la revendication 1 ou 2, *caractérisé* **en ce que** le polynôme $P_a$ comprend le produit $D_{ST} \times D_H$ de la dose mesurée d'analyte $D_{ST}$ par le taux mesuré d'hématocrite $D_H$ ou bien $C_{ST} \times D_H$ de la concentration mesurée en analyte $C_{ST}$ par le taux mesuré d'hématocrite $D_H$.

4. Procédé selon la revendication 4, *caractérisé* **en ce que** le calcul de la dose plasmatique d'analyte est réalisé selon la relation :

$$D_P = a_0 + a_1 \times D_{ST} + a_2 \times D_H + a_{12} \times D_{ST} \times D_H$$

où $a_0$, $a_1$, $a_2$ et $a_{12}$ sont des coefficients prédéterminés, dépendant de l'analyte.

5. Procédé selon la revendication 4, **caractérisé en ce que** le calcul de la concentration plasmatique en analyte est réalisé selon la relation :

$$C_P = a'_0 + a_1 \times C_{ST} + a'_2 \times D_H + a_{12} \times C_{ST} \times D_H$$

où $a'_0$, $a_1$, $a'_2$ et $a_{12}$ sont des coefficients prédéterminés dépendants de l'analyte.

6. Procédé selon la revendication 5, **caractérisé en ce que** l'analyte est un D-dimère, et **en ce que** :

   - $a'_0$ est compris entre -29,311 $\times$ 0,9 et -29,311 $\times$ 1,1;
   - $a_1$ est compris entre 0,788 $\times$ 0,9 et 0,788 $\times$ 1,1;
   - $a'_2$ est compris entre 0,702 $\times$ 0,9 et 0,702 $\times$ 1,1; et
   - $a_{12}$ est compris entre 0,018 $\times$ 0,9 et 0,018 $\times$ 1,1.

7. Procédé selon la revendication 5, **caractérisé en ce que** :

   - $a'_0$ = -29,311 ;
   - $a_1$ = 0,788 ;
   - $a'_2$ = 0,702 ; et
   - $a_{12}$ = 0,018.

8. Procédé selon la revendication 5, **caractérisé en ce que** l'analyte est une troponine I cardiaque, et **en ce que** :

   - $a'_0$ est compris entre -0,0052 $\times$ 0,9 et -0,0052 $\times$ 1,1;
   - $a_1$ est compris entre 0,9155 $\times$ 0,9 et 0,9155 $\times$ 1,1;
   - $a'_2$ est compris entre 0,0002 $\times$ 0,9 et 0,0002 $\times$ 1,1; et
   - $a_{12}$ est compris entre 0,0072 $\times$ 0,9 et 0,0072 $\times$ 1,1.

9. Procédé selon la revendication 7, **caractérisé en ce que** :

   - $a'_0$ = -0,0052 ;
   - $a_1$ = 0,9155 ;
   - $a'_2$ = 0,0002 ; et
   - $a_{12}$ = 0,0072.

10. Procédé selon l'une quelconque des revendications précédentes, **caractérisé en ce que** la mesure de la dose/concentration d'analyte dans le sang total est réalisée selon une technique d'immunodosage de type ELISA, de type ELFA ou de type immuno-capture.

11. Dispositif pour la mesure de la dose plasmatique d'un analyte dans un échantillon de sang total, **caractérisé en ce qu'**il comporte

   - des moyens de réception dudit échantillon de sang total ;
   - des moyens de mesure de la dose d'analyte réalisée sur l'échantillon de sang total ;
   - des moyens de calcul de la dose plasmatique d'analyte selon la relation :

$$D_P = P_a(D_{ST}, D_H)$$

où $D_P$ est la dose plasmatique d'analytes, $D_{ST}$ est la dose d'analyte mesurée, $D_H$ est le taux d'hématocrite

mesuré, et $P_a$ est un polynôme non constant de degré supérieur ou égal à 1 dont les grandeurs indéterminées sont la dose d'analyte mesurée $D_{ST}$ et le taux mesuré d'hématocrite $D_H$, et dont les coefficients polynomiaux dépendent de l'analyte.

**12.** Dispositif pour la mesure de la concentration plasmatique en analyte dans un échantillon de sang total, *caractérisé en ce qu'*il comporte :

- des moyens de réception dudit échantillon de sang total ;
- des moyens de mesure de la dose d'analyte réalisée sur l'échantillon de sang total ;
- des moyens pour transformer la dose d'analyte en concentration ;
- des moyens pour entrer ou pour mesurer le taux d'hématocrite dans l'échantillon de sang total ;
- des moyens de calcul de la concentration plasmatique en analyte selon la relation :

$$C_P = P_a(C_{ST}, D_H)$$

où $C_P$ est la concentration plasmatique en analyte, $C_{ST}$ est la concentration en analyte calculée à partir de la dose mesurée et du volume de l'échantillon, $D_H$ est le taux d'hématocrite mesuré, et $P_a$ est un polynôme non constant de degré supérieur ou égal à 1 dont les grandeurs indéterminées sont la concentration en analyte obtenue à partir de la dose mesurée $C_{ST}$ et le taux mesuré d'hématocrite $D_H$, et dont les coefficients polynomiaux dépendent de l'analyte.

**13.** Dispositif selon la revendication 12, *caractérisé en ce qu'*il est apte à mettre en oeuvre un procédé conforme à l'une quelconque des revendications 3 à 10.


**Patentansprüche**

**1.** Verfahren zur Messung einer im Plasma vorhandenen Menge eines Analyten in einer Vollblutprobe, **dadurch gekennzeichnet, dass** es Folgendes umfasst:

- Messen des Hämatokritwerts der Vollblutprobe;
- Messen einer Analytmenge direkt in der Vollblutprobe; und
- Berechnen der im Plasma vorhandenen Analytmenge gemäß folgender Beziehung:

$$D_P = P_a(D_{ST}, D_H),$$

in der $D_P$ die im Plasma vorhandene Analytmenge ist, $D_{ST}$ die gemessene Analytmenge, $D_H$ der gemessene Hämatokritwert und $P_a$ ein nicht konstantes Polynom vom Grad größer gleich 1, dessen variable Größen die gemessene Analytmenge $D_{ST}$ und der gemessene Hämatokritwert $D_H$ sind und dessen Polynomkoeffizienten vom Analyten abhängig sind.

**2.** Verfahren zur Messung einer Plasmakonzentration eines Analyten in einer Vollblutprobe, **dadurch gekennzeichnet, dass** es Folgendes umfasst:

- Messen des Hämatokritwerts der Vollblutprobe;
- Messen einer Analytmenge direkt in der Vollblutprobe und Umwandeln der gemessenen Menge in eine Konzentration durch Teilen der gemessenen Menge durch das Probenvolumen; und
- Berechnen der Plasmakonzentration des Analyten gemäß folgender Beziehung:

$$C_P = P_a(C_{ST}, D_H),$$

in der $C_P$ die korrigierte Plasmakonzentration des Analyten des Analyten ist, $C_{ST}$ die gemessene Analytmenge, $D_H$ der gemessene Hämatokritwert und $P_a$ ein nicht konstantes Polynom vom Grad größer gleich 1, dessen variable Größen die gemessene Analytkonzentration $C_{ST}$ und der gemessene Hämatokritwert $D_H$ sind und dessen Polynomkoeffizienten vom Analyten abhängig sind.

**3.** Verfahren nach Anspruch 1 oder 2, **dadurch gekennzeichnet, dass** das Polynom $P_a$ das Produkt $D_{ST} \cdot D_H$ aus der gemessenen Analytmenge $D_{ST}$ und dem gemessenen Hämatokritwert $D_H$ oder auch $C_{ST} \times D_H$ aus der gemessenen Analytkonzentration $C_{ST}$ und dem gemessenen Hämatokritwert $D_H$ umfasst.

**4.** Verfahren nach Anspruch 4, **dadurch gekennzeichnet, dass** die Berechnung der im Plasma vorhandenen Analytmenge gemäß folgender Beziehung erfolgt:

$$D_P = a_0 + a_1 \times D_{ST} + a_2 \times D_H + a_{12} \times D_{ST} \times D_H,$$

in der $a_0$, $a_1$, $a_2$ und $a_{12}$ vom Analyten abhängige vorgegebene Koeffizienten sind.

**5.** Verfahren nach Anspruch 4, **dadurch gekennzeichnet, dass** die Berechnung der Plasmakonzentration des Analyten gemäß folgender Beziehung erfolgt:

$$C_P = a'_0 + a_1 \times C_{ST} + a'_2 \times D_H + a_{12} \times C_{ST} \times D_H,$$

in der $a'_0$, $a_1$, $a'_2$ und $a_{12}$ vom Analyten abhängige vorgegebene Koeffizienten sind.

**6.** Verfahren nach Anspruch 5, **dadurch gekennzeichnet, dass** der Analyt ein D-Dimer ist und dadurch, dass:

- $a'_0$ zwischen -29,311 x 0,9 und -29,311 x 1,1 liegt;
- $a_1$ zwischen 0,788 x 0,9 und 0,788 x 1,1 liegt;
- $a'_2$ zwischen 0,702 x 0,9 und 0,702 x 1,1 liegt; und
- $a_{12}$ zwischen 0,018 x 0,9 und 0,018 x 1,1 liegt.

**7.** Verfahren nach Anspruch 5, **dadurch gekennzeichnet, dass**:

- $a'_0$ = -29,311 ;
- $a_1$ =0,788;
- $a'_2$ = 0,702 und
- $a_{12}$ = 0,018.

**8.** Verfahren nach Anspruch 5, **dadurch gekennzeichnet, dass** der Analyt ein kardiales Troponin I ist und dadurch, dass:

- $a'_0$ zwischen -0,0052 x 0,9 und -0,0052 x 1,1 liegt;
- $a_1$ zwischen 0,9155 x 0,9 und 0,9155 x 1;1 liegt;
- $a'_2$ zwischen 0,0002 x 0,9 und 0,0002 x 1,1 liegt; und
- $a_{12}$ zwischen 0,0072 x 0,9 und 0,0072 x 1,1 liegt.

**9.** Verfahren nach Anspruch 7, **dadurch gekennzeichnet, dass**:

- $a'_0$ = -0,0052 ;
- $a_1$ = 0,9155;
- $a'_2$ = 0,0002 und
- $a_{12}$ = 0,0072.

**10.** Verfahren nach einem der vorhergehenden Ansprüche, **dadurch gekennzeichnet, dass** die Messung der Analytmenge/-konzentration im Vollblut nach einem Immunassay-Verfahren vom Typ ELISA, ELFA oder Immunocapture erfolgt.

**11.** Vorrichtung zur Messung der im Plasma vorhandenen Menge eines Analyten in einer Vollblutprobe, **dadurch gekennzeichnet, dass** sie Folgendes umfasst:

- Mittel zum Aufnehmen der Vollblutprobe;
- Mittel zum in der Vollblutprobe erfolgenden Messen der Analytmenge;

- Mittel zum Berechnen der im Plasma vorhandenen Analytmenge gemäß folgender Beziehung:

$$D_P = P_a(D_{ST}, D_H),$$

in der $D_p$ die im Plasma vorhandene Analytmenge ist, $D_{ST}$ die gemessene Analytmenge, $D_H$ der gemessene Hämatokritwert und $P_a$ ein nicht konstantes Polynom vom Grad größer gleich 1, dessen variable Größen die gemessene Analytmenge $D_{ST}$ und der gemessene Hämatokritwert $D_H$ sind und dessen Polynomkoeffizienten vom Analyten abhängig sind.

**12.** Vorrichtung zur Messung der Plasmakonzentration eines Analyten in einer Vollblutprobe, **dadurch gekennzeichnet, dass** sie Folgendes umfasst:

- Mittel zum Aufnehmen der Vollblutprobe;
- Mittel zum in der Vollblutprobe erfolgenden Messen der Analytmenge;
- Mittel zum Umwandeln der Menge in eine Konzentration;
- Mittel zum Eingeben oder Messen des Hämatokritwerts in der Vollblutprobe;
- Mittel zum Berechnen der Plasmakonzentration des Analyten gemäß folgender Beziehung:

$$C_p = P_a(C_{ST}, D_H),$$

in der $C_P$ die Plasmakonzentration des Analyten ist, $C_{ST}$ die Konzentration des Analyten, berechnet aus der gemessenen Menge und dem Volumen der Probe, $D_H$ der gemessene Hämatokritwert und $P_a$ ein nicht konstantes Polynom vom Grad größer gleich 1, dessen variable Größen die aus der gemessenen Menge $C_{ST}$ gewonnene Analytkonzentration und der gemessene Hämatokritwert $D_H$ sind und dessen Polynomkoeffizienten vom Analyten abhängig sind.

**13.** Vorrichtung nach Anspruch 12, **dadurch gekennzeichnet, dass** sie in der Lage ist, ein Verfahren nach einem der Ansprüche 3 bis 10 durchzuführen.

**Claims**

**1.** A method of measuring a plasmatic amount of an analyte in a whole blood sample, *characterized* **in that** it comprises:

- measuring the haematocrit level of the whole blood sample;
- measuring an analyte amount directly in the whole blood sample; and
- calculating the plasmatic amount of the analyte according to relation:

$$D_P = P_a(D_{ST}, D_H)$$

where $D_P$ is the plasmatic amount of the analyte, $D_{ST}$ is the measured analyte amount, $D_H$ is the measured haematocrit level, and $P_a$ is a non-constant polynomial of a degree greater than or equal to 1 having as indeterminate values the measured analyte amount, $D_{ST}$, and the measured haematocrit level, $D_H$, and having its polynomial coefficients depending on the analyte.

**2.** A method of measuring an plasmatic concentration of an analyte in a whole blood sample, *characterized* **in that** it comprises:

- measuring the haematocrit level of the whole blood sample;
- measuring an analyte amount directly in the whole blood sample and transforming the measured amount into a concentration by dividing the measured amount by the sample volume; and
- calculating the plasmitic concentration of the analyte according to relation:

$$C_P = P_a(C_{ST}, D_H)$$

where $C_P$ is the plasmitic concentration of the analyte, $C_{ST}$ is the measured analyte amount, $D_H$ is the measured haematocrit level, and $P_a$ is a non-constant polynomial of a degree greater than or equal to 1 having as indeterminate values the measured analyte concentration, $C_{ST}$, and the measured haematocrit level, $D_H$, and having its polynomial coefficients depending on the analyte.

3.  The device of claim 1 or 2, ***characterized* in that** polynomial $P_a$ comprises product $D_{ST} \times D_H$ of the measured analyte amount $D_{ST}$ by the measured haematocrit level $D_H$, or $C_{ST} \times D_H$ of the measured analyte concentration $C_{ST}$ by the measured haematocrit level $D_H$.

4.  The method of claim 4, ***characterized* in that** the plasmitic amount of the analyte is calculated according to relation:

$$D_P = a_0 + a_1 \times D_{ST} + a_2 \times D_H + a_{12} \times D_{ST} \times D_H$$

where $a_0$, $a_1$, $a_2$ and $a_{12}$ are predetermined coefficients depending on the analyte.

5.  The method of claim 4, ***characterized* in that** the plasmitic concentration of the analyte is calculated according to relation:

$$C_P = a'_0 + a_1 \times C_{ST} + a'_2 \times D_H + a_{12} \times C_{ST} \times D_H$$

where $a'_0$, $a_1$, $a'_2$ and $a_{12}$ are predetermined coefficients depending on the analyte.

6.  The method of claim 5, ***characterized* in that** the analyte is a D-dimer, and **in that**:

    - $a'_0$ is in the range from -29.311 $\times$ 0.9 to -29.311 $\times$ 1.1;
    - $a_1$ is in the range from 0.788 $\times$ 0.9 to 0.788 $\times$ 1.1;
    - $a'_2$ is in the range from 0.702 $\times$ 0.9 to 0.702 $\times$ 1.1; and
    - $a_{12}$ is in the range from 0.018 $\times$ 0.9 to 0.018 $\times$ 1.1.

7.  The method according to claim 5, ***characterized* in that**:

    - $a'_0$ = -29.311 ;
    - $a_1$ = 0.788;
    - $a'_2$ = 0.702 ; and
    - $a_{12}$ = 0.018.

8.  The method of claim 5, ***characterized* in that** the analyte is a cardiac troponin I, and **in that**:

    - $a'_0$ is in the range from -0.0052 $\times$ 0.9 to -0.0052 $\times$ 1.1;
    - $a_1$ is in the range from 0.9155 $\times$ 0.9 to 0.9155 $\times$ 1.1;
    - $a'_2$ is in the range from .0002 $\times$ 0.9 to 0.0002 $\times$ 1.1; and
    - $a_{12}$ is in the range from 0.0072 $\times$ 0.9 to 0.0072 $\times$ 1.1.

9.  The method according to claim 7, ***characterized* in that**:

    - $a'_0$ = -0.0052 ;
    - $a_1$ = 0.9155;
    - $a'_2$ = 0.0002; and
    - $a_{12}$ = 0.0072.

10. The method of any of the foregoing claims, ***characterized* in that** the analyte amount/concentration in the whole blood sample is measured according to an immunoassay technique of ELISA type, of ELFA type, or of immunocapture type.

11. A device for measuring the plasmatic amount of an analyte in a whole blood sample, ***characterized* in that** it comprises

- means for receiving said whole blood sample;
- means for measuring the total analyte amount in the whole blood sample;
- means for calculating a corrected analyte amount according to relation:

$$D_P = P_a(D_{ST}, D_H)$$

where $D_P$ is the plasmitic amount of the analyte, $D_{ST}$ is the measured analyte amount, $D_H$ is the measured haematocrit level, and $P_a$ is a non-constant polynomial of a degree greater than or equal to 1 having as indeterminate values the measured analyte amount, $D_{ST}$, and the measured haematocrit level, $D_H$, and having its polynomial coefficients depending on the analyte.

12. A device for measuring the plasmatic analyte concentration in a whole blood sample, ***characterized* in that** it comprises

- means for receiving said whole blood sample;
- means for measuring the total analyte amount in the whole blood sample;
- means for transforming the analyte amount into a concentration;
- means for inputting or for measuring the haematocrit level in the whole blood sample;
- means for calculating the plasmitic concentration of the analyte according to relation:

$$C_P = P_a(C_{ST}, D_H)$$

where $C_P$ is the plasmitic concentration of the analyte, $C_{ST}$ is the analyte concentration calculated from the measured amount and from the sample volume, $D_H$ is the measured haematocrit level, and $P_a$ is a non-constant polynomial of degree greater than or equal to 1 having as indeterminate values the analyte concentration obtained from the measured amount, $C_{ST}$, and the measured haematocrit level, $D_H$, and having its polynomial coefficients depending on the analyte.

13. The device of claim , ***characterized* in that** it is capable of implementing the method of any of claims 3 to 10.

Fig. 1A

Fig. 1B

Fig. 1C

Fig. 1D

Fig. 1E

Fig. 1F

Fig. 1G

**Fig. 2**

Concentration_sangtotal (ng/ml)

● Etablissement du modèle    hématocrite (%)
☆ Vérification du modèle

**Fig. 3**

Scatter Plot with Passing & Bablok Fit

concentration
sang total (ng/ml)

**Fig. 4**

- - - Identity
——— Passing & Bablock (I) fit
(-8,80 + 0,69x)

Concentration plasma (ng/mL)

**Fig. 5**

**Fig. 6**

Scatter Plot with Passing & Bablok Fit

**Fig. 7**

**Fig. 8**

Scatter Plot with Passing & Bablok Fit

Fig. 9

Vérification du modèle mathématique (sans les échantillons avec dose sang total >= dose plasma)

Fig. 10

Scatter Plot with Passing & Bablok Fit

Fig. 11

Scatter Plot with Passing & Bablok Fit

Fig. 12